**Europäisches Patentamt**

(19) **European Patent Office**

**Office européen des brevets**

(11) Publication number : **0 337 757 B1**

(12) **EUROPEAN PATENT SPECIFICATION**

(45) Date of publication of patent specification :
26.02.92 Bulletin 92/09

(51) Int. Cl.⁵ : **A61F 2/36**

(21) Application number : **89303611.1**

(22) Date of filing : **12.04.89**

(54) Endoprostheses with bone resorption preventing means.

(30) Priority : **12.04.88 US 180467**

(43) Date of publication of application :
**18.10.89 Bulletin 89/42**

(45) Publication of the grant of the patent :
**26.02.92 Bulletin 92/09**

(84) Designated Contracting States :
**AT BE CH DE ES FR GB GR IT LI LU NL SE**

(56) References cited :
**EP-A- 0 225 838**
**FR-A- 2 404 429**
**GB-A- 2 118 441**
**US-A- 3 992 725**
**US-A- 4 642 124**

(73) Proprietor : **TRANQUIL PROSPECTS LTD.**
**P.O. Box 47, McNamara Chambers**
**Road Town, Tortola (VG)**

(72) Inventor : **Homsy, Charles A.**
**11526 Raintree Circle**
**Houston Texas 77024 (US)**

(74) Representative : **Lambert, Hugh Richmond et al**
**D. YOUNG & CO. 10 Staple Inn**
**London, WC1V 7RD (GB)**

# Description

The invention relates, in general, to endoprostheses of the type which include a stem for insertion into a canal of a bone and, in particular, into the medullary canal of a femur. An endoprothesis according to the preamble of Claim 1 is known from US-A- 4 642 124.

Endoprostheses include implants, which rely on an accurate press-fit to become established between an uncoated stem and surrounding bone of the femur, which rely on tissue ingrowth into a porous soft coating that covers the entire stem, and which rely, in part, on a postoperative biological bond to become established between surrounding bone and a hard porous surface on the proximal stem.

Initially, these hard surface porosities were porous coatings bonded to the entire external surface of the stem. It was found that bone would bond to apposite hard porous coating. While such bonding is desired for the proximal stem, it can be very detrimental to the distal femur.

In more recent hip-joint endoprostheses, and in an effort to prevent this distal bond, hard surface porosity is confined mostly to the proximal stem, while the distal stem surface is purposely left bare.

Unfortunately, clinical evidence has shown that the various attempts to use hard surface porosities in order to achieve long-lasting, adequate, proximal load transfer, as well as proximal implant stability and fixation, have not been entirely satisfactory. It was found that a thin bone phase forms externally on the distal femur, and a dense and thick bone forms under the stem's tip within the medullary canal.

The formation of such a dense and thick bone under the stem's tip increasingly shunts load from proximal femur to distal femur, in the region apposite to the stem's tip, which leads to a gradually increasing proximal bone resorption, which reduces the proximal femur's resistance to implant rotation, and which further accelerates bone resorption. All of which unavoidably leads to implant instability, great discomfort, and severe pain to the patient.

Even an endoprosthesis having a soft, tissue ingrowth promoting coating, over the stem's entire external surface, has been found to also experience a thin bone growth around the stem's tip.

In this invention means are provided substantially to eliminate or attenuate the transfer of cyclic axial and rotational stresses from the distal stem of an endoprosthesis to apposite cortical bone of the femur in the diaphyseal region. More particularly, in accordance with this invention, an energy-absorbing member is provided on and/or around the distal tip of the stem of the prosthesis. In one embodiment of the endoprosthesis, the energy-absorbing member extends distally from the stem's tip. The member can be a soft, porous pad having a thickness greater than two millimeters. In another embodiment, the stem's distal region is also biocompatible, porous, soft, resilient. deformable, and tissue-ingrowth promoting. In a further embodiment, the stem's surface is also substantially entirely biocompatible, porous, soft, resilient, deformable, and tissue-ingrowth promoting. In yet another embodiment, at least a portion of the stem's proximal surface is also biocompatible, porous, deformable, and tissue-ingrowth promoting, while another portion of the proximal surface is biocompatible, porous, nondeformable, and tissue-ingrowth promoting.

The invention is further described in connection with accompanying drawings, wherein:

FIG.1 is an elevational view of a known uncoated hip endoprosthesis within a femur, in which the socket under the stem's distal tip is nearly totally plugges up by inward bone growth, and in which the bone resorption process has already well progressed;

FIG. 2 is a sectional view taken on line 2-2 of FIG. 1;

FIG. 3 is an enlarged elevational view of distal femur of FIG. 1, showing the socket under the stem's tip to be already totally plugged up, and further showing external bone growth on the distal femur;

FIG. 4 is a sectional view taken on line 4-4 of FIG. 3;

FIG. 5 is an elevational view of uncoated endoprosthesis of FIG. 1 utilizing a simple embodiment of the energy-absorbing, soft, porous, biocompatible member of the invention;

FIG. 6 is a perspective view of the member shown in FIG. 5;

FIG. 7 is an elevational view of a known endoprosthesis that is fully coated with porous metal on the surface of its proximal stem, and which utilizes an improved embodiment of the invention that includes a pad and also a soft, porous, biocompatible distal sleeve or coating extending upwardly from the pad on the bare surface of the stem;

FIG. 8 is an elevational view of a known endoprosthesis coated with discrete porous metal patches on the surface of its proximal stem and which utilizes the pad and the sleeve of the invention;

FIG. 9 is an elevational view of a known endoprosthesis whose stem is coated with a very soft porous layer, shown in section, and which utilizes below the stem's tip the energy-absorbing member of the invention;

FIGS. 10 and 11 are sectional views on lines 10-10 and 11-11 of FIG. 9, respectively;

FIG. 12 is an elevational view of a novel endoprosthesis coated with discrete porous metal patches on the stem's proximal surface; the stem has a soft, porous, biocompatible sleeve or coating over its entire bare surface and the energy-absorbing member below its distal tip;

FIG. 13 is a sectional view taken on line 13-13 of FIG. 12;

FIG. 14 is a sectional view, similar to FIG. 13, but showing the stem within its socket in the femur; and

FIG. 15 is a sectional view, similar to FIG. 13, but showing the porous metal coating as being recessed from the stem's surface.

To simplify the description, the invention will be described using the same numerals, whenever possible, to designate the same or similar parts.

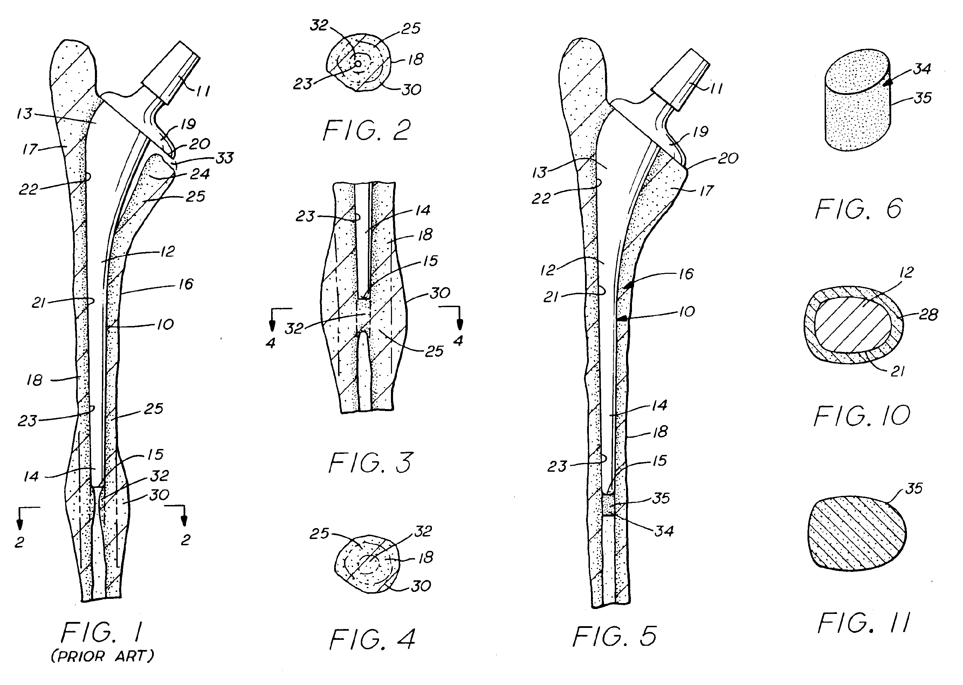

The severe problems solved by the invention will become more apparent from a brief description of a known hip-joint endoprosthesis 10 (FIG. 1-4), shown in position with a preformed socket within the femur 16, which has a proximal region 17 and a distal region 18. Implant 10 includes a neck 11 and a stem 12 that has a proximal exterior or outside region 13, a distal exterior or outside region 14, and a distal tip 15. As used herein in respect to stem 12, "region" may be a three-dimensional locus of points which has depth. For example, stem's distal region 14 can be a layer, a coating, or the stem's exterior surface.

If accurately fitted, stem 12 can transfer appropriate stresses to femur 16. The accuracy of fit, however, is in practice very difficult to achieve. Therefore, some stems 12 are additionally provided with collars 19 to help them to transmit load to superior surface 20 of the cut femoral neck.

The medullary canal in proximal femur 17 has a complex sectional shape whose major axis is in the medial-lateral direction. The canal's sectional shape gradually changes to increasingly circular in the diaphyseal region of distal femur 18. More distally, near circular sectional shape can again change to the complex shape, but this time its major axis will be in the anterior/posterior direction.

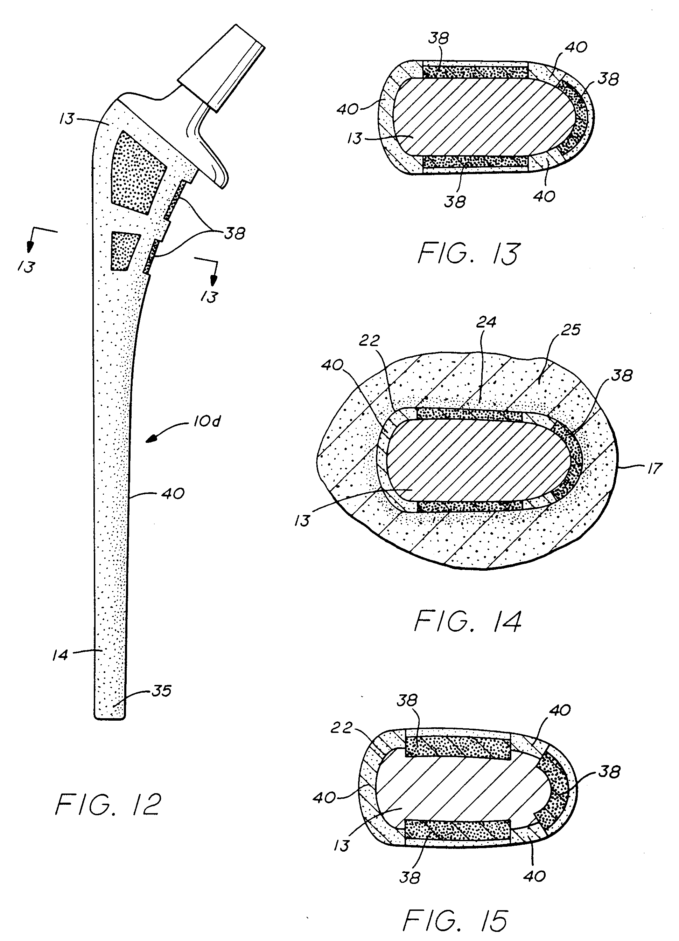

Within medullary canal, the surgeon forms a socket 21, which has a proximal region 22 and a distal region 23. Socket 21 is defined by hard cancellous bone 24 (FIG. 14) and by dense compact cortical bone 25 in the epiphyseal and metaphyseal regions, following removal of softer cancellous bone from within these regions. In the diaphyseal region, socket 21 is defined only by cortical bone 25 (FIG. 4).

In an attempt to avoid intruding into viable bone, stem 12 is shaped to seat centrally in and follow the natural contours of socket 21. To mimic the contour and shape of proximal stem 13 (FIG. 13), the sectional shape of proximal socket 22 is generally trapezoidal, but with rounded corners, whose major axis is in the medial/lateral direction and, in distal socket 23 (FIG. 1), the sectional shape gradually changes and becomes increasingly circular but of reduced area.

For use with uncoated implant 10 without cement or grout, the surgeon generally shapes socket 21 to conform as closely as possible to the geometrical shape of stem 12. He intends thereby to obtain, intraoperatively, a generally uniform press fit between stem 12 and appositional hard cancellous and cortical bones 24,25, respectively.

If a perfect press-fit is achieved, prepared socket 21 will have, at each point over substantially its entire length, a transverse sectional area which corresponds substantially with corresponding transverse sectional area of stem 12 at that point.

An implant 10 which combines a uniform press-fit between proximal stem 13 and appositional hard cancellous bone 24, and which, in addition has a collar 19 that is shaped to precisely match apposite cut surface 20 on the femoral neck, can properly transfer loads to proximal femur 17. Such load transfer will develop strains in the proximal femur that are similar to those induced by normal hip-joint movements within an intact femur. In practice, however, such a press-fit and perfect collar match are very difficult to achieve during operation.

In use of implant 10, resistance to stem rotation is in large part the result of keying the sectional shape of proximal stem 13 (FIG. 14) to a corresponding sectional shape of proximal socket 22.

In response to hip joint loads produced by normal ambulation, proximal stem 13 distributes stresses to proximal femur 17, circumferentially as tensile hoop stresses, longitudinally as compressive medial stresses, and laterally as tensile stresses.

Distal stem 14 (FIGS. 1-4) and/or its stem tip 15 transfer corresponding stresses and strains to apposite cortical bone 25 in distal femur 18. Distal femur 18 may respond to received stresses by enlarging its bone structure, which can manifest itself an an external boss 30 on distal femur 18, and as an internal bridge 32 under stem's tip 15.

Such bone formation is believed to be prompted by relatively intense stresses which are transmitted, at least in part, through stem's tip 15 to apposite bone. Such stresses are believed to be caused by axial bending and torsional cyclic forces, acting about long axis of stem 12 in response to normal hip-joint ambulation.

In FIGS. 1 and 2, bridge 32 is shown as being nearly fully developed. When bridge 32 is fully formed (FIGS. 3-4), it effectively plugs up distal socket 23 below stem's tip 15, and it now has necessary strength to fully react against physiological ambulation loads transmitted to and/or through stem's tip 15.

The more reaction forces that are provided by bridge 32, the less reaction forces will be needed from proximal femur 17. This principle is known as "load shunting" from proximal femur 17 to distal femur 18.

The gradual, osseous resorption, generally designated as 33 (FIG. 1), which takes place within proximal femur 17, especially on its anterior, posterior and medial aspects, is believed to be the result of the transfers by distal stem 14, and/or by stem's tip 15, of stresses and strains to apposite cortical bone 25 in distal femur 18, and the consequential formation of bridge 32.

As decalcification of osseous resorption 33 increases, proximal femur 17 becomes less and less able to resist the dangerous and increasing tendency of proximal stem 13 to rotate within proximal socket 22, as well as the tendency by stem tip 15 to toggle. All of which further prompts femur 16 to stimulate osseous production under stem's tip 15, which further accelerates proximal osseous resorption already in progress until, sooner or later, a sufficient loosening of stem 12 within its socket 21 takes place, which is accompanied by great pain and extraordinary discomfort to the patient.

In order to eliminate or substantially reduce the femur's bone resorption, there is provided, in accordance with this this invention, an energy absorbing member 34 which is designed to substantially absorb mechanical energy that is being transferred to and/or through distal stem 14 and/or its tip 15, or at least to largely attenuate the intensity of such loads that do transfer, to apposite cortical bone 25 in diaphyseal region 18 of femur 16.

The present invention has utility for hip joints as well as for other body joints, for uncoated endoprostheses, for endoprostheses whose stems receive hard surface porosities, as well as those that receive soft surface porosities, and combinations thereof.

Implant 10 (FIGS. 1-6) has an uncoated stem 12 which is associated, in accordance with a simple embodiment of this invention, with an energy-absorbing member, generally designated as 34, and which can be a pad 35. It is shown disposed adjacent and opposite to stem's tip 15 (FIG. 5). Preferably, it is bonded to stem's tip 15 (FIGS. 6-9 and 11-12).

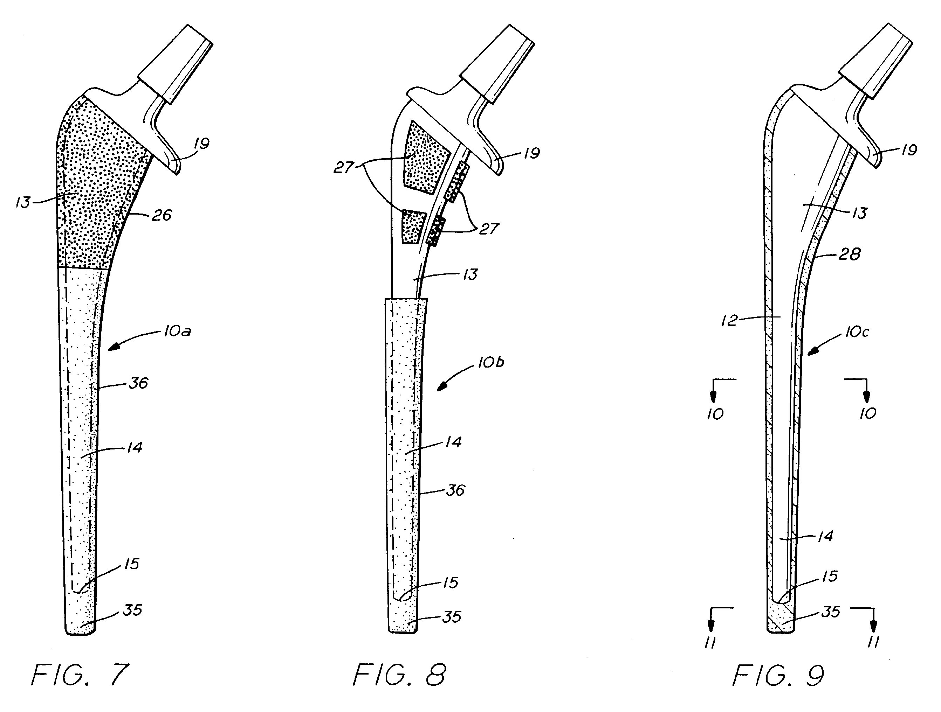

Implant 10a (FIG. 7) has a proximal stem region 13 which is coated with a continuous thin, hard, porous coating 26. In this embodiment of the invention, energy-absorbing member 34, in addition to pad 35, also includes a soft, porous, distal sleeve or coating 36 extending upwardly from and being continuous with pad 35. Coating 36 is applied to bare metal of stem 12 in a suitable manner. Soft sleeve 36 is confined mainly to stem's distal region 14.

Pad 35 and sleeve 36 serve to absorb or largely attenuate longitudinal, lateral, and rotational stresses and micromotions transferred to distal stem 14.

Such mechnical energy absorption or attenuation, in the region appositional to stem's tip 15, prevents unnatural load shunting to, and reduces localized stress zones in, distal femur 18. Such prevention allows stem' proximal region 13 to maintain its load transfer capabilities, thereby ensuring osseous integrity of proximal femur 17, all of which foster favorable conditions for long-term fixation of proximal stem 13.

Implant 10b (FIG. 8) has a proximal stem region 13, which is coated with discrete, hard-porous patches 27, and a distal stem region 14, which has pad 35 and sleeve 36.

Implants 10a and 10b are implanted without use of cement or grout. It is intended that when proximal stem 13 is inserted into proximal socket 22, a press-fit will form, at least between the hard coatings and apposite hard cancellous bone 24. To allow for a press-fit of soft coating 36, socket 21 is made to have, at each point adjacent to soft coating 36, a transverse sectional area which is slightly less than the corresponding transverse sectional area of soft coated stem 12 at that point.

At the junction between hard and soft coatings on the stem of implant 10a, soft-coated cross-sections will be larger than adjacent hard-coated cross-sections, so that following compression of soft coating, hard coating will have a press-fit against adjacent hard cancellous bone 24 and/or cortical bone 25.

Implant 10c (FIGS. 9-11) has a stem 12 which is substantially-fully coated with a very soft, tissue-ingrowth promoting, very porous coating 28 over its entire bare outer surface. The stem's distal region 14 of implant 10c received pad 35 below stem's tip 15. Implant 10c is more fully described in U.S. patent 4,636,214.

Implant 10d (FIGS. 12-14) has discrete, hard-porous patches 38 on stem's proximal region 13. The entire remainder of bare outer surface of stem 12 is coated with a soft, porous coating 40. Stem's distal region 14 also received pad 35 below stem tip 15.

To use implants 10c and 10d, the surgeon shapes socket 21 to conform, as perfectly as possible, to the geometric shape of stem 12. To allow for compression of soft coating 28 or soft coating 40, socket 21 is made to have, at each point over substantially its entire length, a transverse sectional area which is slightly less than the corresponding transverse sectional area of coated stem 12 at that point.

The soft porous coating must compress, at least by a sufficient amount, so as to reduce the overall dimension of the coated stem to that of the porous metal (compare FIGS. 13 and 14).

When coated stem 12 is forcefully inserted into socket 21, each soft porous coating 28, 36 or 40 becomes compresses (FIG. 13) and provides a desired press-fit, thereby achieving an optimal initial stability and an extensive surface contact area between the compressed porous coating and appositional hard cancellous and cortical bones 24,25, respectively.

A compression of the soft coating by about 1 to 40% will bring the hard-porous metal coating into apposition with adjacent bone, thereby maximizing the possibility of bone ingrowth into porous metal. At the same time, such biological bonding with hard coating will be augmented by fibrous tissue and/or bone ingrowth into the soft deformed coating.

The compression is preferably such that at least the medial-lateral sectional shape of coated proximal stem 13, at any given point, is slightly larger than the corresponding medial-lateral dimension of proximal socket 22 at that point, whereby, when stem 12 is inserted into socket 21, soft porous coating 28 or 40 becomes compressed, at least in the medial/lateral direction, between the core of stem 12 and appositional cancellous bone 24 or cortical bone 25, but without substantially restricting the porosity of coating 40.

The opportunity for tissue ingrowth into soft and hard coatings provides an optimal initial and a long-term stability, as well as a distributed, rather than a multi-point, biological fixation.

In addition, if collar 19 is precisely shaped to match appositional surface 20 of the cut femoral neck then, postoperatively, implants 10, 10a-10d will be able to transfer loads to cut surface 20, similar to loads produced by normal hip-joint movements in an intact femur.

The convalescing patient can immediately impose postoperatively a cyclic load on proximal femur 17. This load is analogous to the load imposed on a natural, unoperated proximal femur 17.

Implants 10 and 10a-10d are typically made of a strong, hard, biocompatible metal, such as stainless steel, cobalt-chrome, or titanium. They may also be made of a sufficiently-strong, composite, biocompatible structure of metal with polymers, or of wholly polymeric structures, which may be reinforced with metal or ceramic or other materials, such as polyimide fiber or carbon fiber. If the implant is uncoated (FIG. 5), new bone will grow up and then interface and adapt to stem 12.

The biocompatible hard, tissue-ingrowth promoting, surface porosities 26, 27 and 38 can be ceramic, polymeric, metallic or a composite material thereof. These surface porosities can be either wholly or partically of a homogeneous porous phase, or they can be combinations of different porous phases. They can be made of porous titanium or of other biocompatible metal, and such metal porosities can in turn be coated with synthetic hodroxylapatite.

A postoperative mechanical bond is formed between living bone and hard-porous coated surfaces. This bond is known as biointegration. The hard-porous coatings can also be designed to form a post operative, mechanical and biochemical bond with living bone. This bond is known as osseo-integration.

Hard coating 26 of implant 10a, hard patches 27 of implant 10b, and hard patches 38 of implant 10d can be on the surface of proximal stem 13 (FIG. 13), or they can be countersunk into the stem's body (FIG. 15). Patches 17 on implant 10b and patches 38 on implant 10d are preferably disposed on the anterior, posterior and medial aspects of proximal stem 13.

The material out of which pad 35 and soft coatings 28, 36 and 40 are made should, preferably, have a three-dimensional structure, characterized by at least a low-modulus of elasticity, a continuous biocompatible solid phase, and a biocompatible fluid phase.

In the preferred embodiment, the fluid phase is continuous with the ambient environment. This preferential structure should exhibit a compressive stress-strain behavior of a substantially lower order than cortical or cancellous bone. That is, this structure must deform under load much more readily than the appositional bone.

The material for pad 35 and for the soft coatings can have an open pore size of 50 to 400 micro-millimeters which has been found to be optimal. A material that meets the above criteria can be elastomeric or nonelastomeric, resilient, open or closed pore, and very soft, so that sufficient deformation in its shape can occur in response to loading by distal stem 14, as well as by any subsidence of stem 12.

This soft porous material for pad 35 should become compressed in response to an initial loading, but without substantially restricting its porosity, thereby permitting a subsequent compression to take place. Such a material will be nonstimulating to cortical bone 25 in distal femur region 18.

The porosity of pad 35 will allow ingrowth therein of fibrous tissue, which will further enhance its ability to attenuate or absorb energy transferred to distal stem 14.

Pad 35 has a porosity such that its volume can shrink up to 60-80% of its original volume before substantial resistance to compression becomes manifest.

Pad 35 (FIG. 6) can be a short cylinder having an outer diameter, which is slightly less than the smallest diameter of socket 21, in order to facilitate insertion of stem 12 therein, or it can be slightly larger than the diameter of distal socket 23 to obtain a pressure fit with appositional hard cortical bone 25.

Pad 35 can have a trapezoidal sectional shape with rounded corners and with the main axis in the anterior-posterior direction to better fit the corresponding shape of distal socket 23.

It has been found that the thickness for the material from which pad 35 is made, between its interior surface apposite to stem tip 15 and its outside surface, should be greater than 2mm and, preferably, greater than 4mm to provide adequate biomechanical energy absorption.

Where the hard coatings are located, the diametrical dimensions correspond to those of the opposite proximal femur socket.

The soft coatings may have a variable thickness at any given cross-section of the stem so that they will be thicker in the anterior-posterior direction to better fit the sectional shape of distal socket 23.

The thickness of the resilient and deformable soft coatings may be such that the overall dimensions of the coated stem will exceed the corresponding dimensions of prepared socket 21 by an amount ranging from 0.2% to 10%.

It has been found that this thickness for the material from which the soft coatings are made should be greater than .5mm, and preferably greater than 1.5mm and less than 2.5mm.

The thickness of the soft coatings is such that they may be compressed by an amount ranging from one percent (1%) to forty percent (40%) of their original thickness without having their porosities overly reduced.

The material out of which pad 35 and soft coatings 28, 36 and 40 are made can be a porous Teflon perfluorocarbon polymer. Such materials, including tissue-ingrowth promoting additives, are sold by Novamed, Inc., Houston, Texas, USA, under the trademark PROPLAST.

A PROPLAST material is a reinforced polymer which comprises polytetrafluoroethylene fibers, in admixture with a proportion of carbon or graphite fiber or particles, or aluminum oxide particles and bonded with a sintered polytetrafluoroethylene resin. PROPLAST materials are soft, very porous, open pore, ingrowth-promoting, compressible, and permanently deformable under very light loads.

This invention fills the need for more natural stress and strain patterns within femur 16 by optimizing the conditions for intimate engagement with appositional bone. The invention allows for a more stable apposition of porous metal to adjacent bone, thereby achieving an improved distributed, longitudinal-load transfer to femur 16. The forces per unit of surface area become largely reduced.

By immediately imposing, post operatively, a cyclic load on proximal femur 17, analogous to the load of a natural, unoperated femur, proximal femur 17 responds and maintains maximal strength and resistance against rotation by stem 12.

Thus, the surgeon can now select from a range of different endoprostheses including those which rely on an accurate press-fit to become established between an uncoated stem and surrounding bone, and those which rely on tissue ingrowth into a porous coating on the stem's outer surface. The surgeon can choose also from implants which are uncoated, or coated with a very-soft porous material, or coated with a hard-porous material, or with combinations thereof.

## Claims

1. An endoprosthesis for insertion into a socket (21) formed within the canal of a patient's bone (16), comprising an elongated stem (12) defining a proximal region (13), a distal region (14) and a distal tip (15) which is provided with an energy absorbing member (34) of soft, porous, biocompatible, resilient and deformable material extending therefrom, characterised in that the material is a tissue ingrowth promoting material having a thickness measured in the axial direction of the prosthesis greater than two millimetres.

2. An endoprosthesis according to Claim 1, characterised in that said member is attached to the distal end (15) of the prosthesis.

3. An endoprosthesis according to Claim 1 or 2, characterised in that said member (34) has a three-dimensional structure having a low-modulus of elasticity, a continuous biocompatible solid phase, and a biocompatible fluid phase.

4. An endoprosthesis according to Claim 3, characterised in that said biocompatible fluid phase is continuous with the ambient environment.

5. An endoprosthesis according to Claim 3 or 4, characterised in that said member (34) has a compressive, stress-strain behavoir of a substantially lower order than that of cancellous bone (24).

6. An endoprosthesis according to any one of Claims 1 to 5, characterized in that said material has an open pore size of 50 to 400 micro-millimeters.

7. An endoprosthesis according to any one of Claims 1 to 6, characterised in that said member has a thickness greater than 4mm.

8. An endoprosthesis according to any one of Claims 1 to 7, characterised in that said material has a porosity such that the volume of said member can shrink up to 60-80% of its original volume before substantial resistence to compression becomes manifest.

9. An endoprosthesis according to any one of Claims 1 to 8, characterised in that said material is elastomeric.

10. An endoprosthesis according to any one of Claims 1 to 8, characterised in that said material is nonelastomeric.

11. An endoprosthesis according to any one of Claims 1 to 8, characterised in that said material comprises polytetrafluoroethylene fibers, in admixture with carbon fiber, graphite or aluminium oxide and bonded with a sintered polytetrafluoroethylene resin.

12. An endoprosthesis according to any one of Claims 1 to 11, characterised in that the energy-absorbing member (34) is in the form of a pad (35) affixed to the distal end (15) of the stem (12) of the prosthesis.

13. An endoprosthesis according to any one of Claims 1 to 11, characterised in that the energy-absorbing member (34) is integral with a sleeve (36) mounted on the stem (12) of the prosthesis and extending over the end (15) of the prosthesis.

14. An endoprosthesis according to any one of preceding claims, characterised in that at least a portion of the proximal region (13) of the stem (12) of the prosthesis is coated with a layer of biocompatible, porous, non-deformable and tissue-ingrowth promoting material (26, 27, 38).

15. An endoprosthesis according to Claim 14, characterised in that said portion is disposed on the anterior aspect of the proximal region (13) of the stem (12).

16. An endoprosthesis according to Claim 14 or 15, characterised in that in at least one other portion of the stem proximal region (13) of the stem (12) is coated with a biocompatible, porous, deformable, and tissue-ingrowth promoting material (28, 36, 40).

17. An endoprosthesis according to any one of Claims 1 to 16, characterised in that at least a portion of the distal region (14) of the stem (12) is coated with a biocompatible, porous, soft, resilient, deformable, and tissue-ingrowth promoting material.

18. An endoprosthesis according to Claim 14, 15 or 16, characterised in that apart from the regions coated with said non-deformable material (26, 27, 38) substantially the whole of the remainder of said stem (12) is coated with a biocompatible, porous, soft, resilient, deformable, and tissue-ingrowth promoting material.

19. An endoprosthesis according to any one of Claims 14 to 18, characterised in that said coatings have a thickness less than 2.5mm.

20. An endoprosthesis according to any one of Claims 16 to 19, characterised in that the deformable coating layer has resiliency such that it can be compressed by an amount ranging from one percent (1%) to forty percent (40%) of its original thickness.

21. An endoprosthesis according to any one of Claims 14 to 20, characterised in that the coating layers have an open pore size of 50 to 400 micro-millimeters.

**Patentansprüche**

1. Endoprothese zum Einfügen in eine Hülse (21), die in dem Kanal eines Patientenknochens (16) ausgebildet ist, mit einem länglichen Stiel (12), der einen proximalen Bereich (13), einen distalen Bereich (14) und eine distale Spitze (15) definiert, welche mit einem Energie absorbierenden Teil (34) aus weichem, porösem, biologisch verträglichem, elastischem und deformierbarem Material, welches sich von dort erstreckt, versehen ist, **dadurch gekennzeichnet, daß das Material ein Gewebeeinwuchs förderndes Material mit einer Dicke, gemessen in der Axialrichtung der Prothese, größer als 2 mm ist.

2. Endoprothese nach Anspruch 1, **dadurch gekennzeichnet, daß das Teil an dem distalen Ende (15) der Prothese befestigt ist.

3. Endoprothese nach Anspruch 1 oder 2, **dadurch gekennzeichnet, daß das Teil (34) eine** dreidimensionale Struktur mit einem niedrigen Elastizitätsmodul, einer zusammenhängenden biologisch verträglichen festen Phase und einer biologisch verträglichen Fließmittelphase hat.

4. Endoprothese nach Anspruch 3, **dadurch gekennzeichnet, daß die biologisch verträgliche Fließmittelphase sich in der Umgebung fortsetzt.

5. Endoprothese nach Anspruch 3 oder 4, **dadurch gekennzeichnet, daß das Teil (34) ein kompressives Belastungs-Verformungsverhalten von wesentlich geringerer Größenordnung als jenes von schwammig-porigem Knochen (24) hat.

6. Endoprothese nach einem der Ansprüche 1 bis 3, **dadurch gekennzeichnet, daß das Material eine offene Porengröße von 50 bis 400 Mikromillimeter besitzt.

7. Endoprothese nach einem der Ansprüche 1 bis 6, **dadurch gekennzeichnet, daß das Teil eine größere Dicke als 4 mm hat.

8. Endoprothese nach einem der Ansprüche 1 bis 7, **dadurch gekennzeichnet, daß das Material eine solche Porosität hat, daß das Volumen des Teils bis zu 60 bis 80 % seines ursprünglichen Volumens schrumpfen kann, bevor ein wesentlicher Widerstand gegen Zusammendrücken auftritt.

9. Endoprothese nach einem der Ansprüche 1 bis 8, **dadurch gekennzeichnet, daß das Material elastomer ist.

10. Endoprothese nach einem der Ansprüche 1 bis 8, **dadurch gekennzeichnet, daß das Material nicht elastomer ist.

11. Endoprothese nach einem der Ansprüche 1 bis 8, **dadurch gekennzeichnet, daß das Material Polytetrafluorethylenfasern im Gemisch mit Kohlenstoffasern, Graphit oder Aluminiumoxid und mit einem gesinterten Polytetrafluorethylenharz gebunden umfaßt.

12. Endoprothese nach einem der Ansprüche 1 bis 11, **dadurch gekennzeichnet, daß das Energie absorbierende Teil (34) in der Form eines Kissens (35) vorliegt, das an dem distalen Ende (15) des Stiels (12) der Prothese befestigt ist.

13. Endoprothese nach einem der Ansprüche 1 bis 11, **dadurch gekennzeichnet, daß das Energie absorbierende Teil (34) aus einem Stück mit einer Hülse (36) besteht, die auf dem Stiel (12) der Prothese angeordnet ist und sich über das Ende (15) der Prothese hinaus erstreckt.

14. Endoprothese nach einem der vorausgehenden Ansprüche, **dadurch gekennzeichnet, daß wenigstens ein Teil des proximalen Bereiches (13) des Stiels (12) der Prothese mit einer Schicht eines biologisch verträglichen, porösen, nicht deformierbaren und Gewebeeinwuchs fördernden Materials (26, 27, 38) überzogen ist.

15. Endoprothese nach Anspruch 14, **dadurch gekennzeichnet, daß dieser Teil auf der vorderen Seite des proximalen Bereiches (13) des Stiels (12) angeordnet ist.

16. Endoprothese nach Anspruch 14 oder 15, **dadurch gekennzeichnet**, daß wenigstens ein anderer Teil des proximalen Bereiches (13) des Stiels (12) mit einem biologisch verträglichen, porösen, deformierbaren und Gewebeeinwuchs fördernden Material (28, 36, 40) überzogen ist.

17. Endoprothese nach einem der Ansprüche 1 bis 16, **dadurch gekennzeichnet**, daß wenigstens ein Teil des distalen Bereiches ( 14) des Stiels (12) mit einem biologisch verträglichen, porösen, weichen, elastischen, deformierbaren und Gewebeeinwuchs fördernden Material überzogen ist.

18. Endoprothese nach Anspruch 14, 15 oder 16, **dadurch gekennzeichnet,** daß abgesehen von den mit dem nichtdeformierbaren Material (26, 27, 38) überzogenen Bereichen im wesentlichen der gesamte Rest des Stiels (12) mit einem biologisch verträglichen, porösen, weichen, elastischen, deformierbaren und Gewebeeinwuchs fördernden Material überzogen ist.

19. Endoprothese nach einem der Ansprüche 14 bis 18, **dadurch gekennzeichnet**, daß die Überzüge eine Dicke von weniger als 2,5 mm haben.

20. Endoprothese nach einem der Ansprüche 16 bis 19, **dadurch gekennzeichnet**, daß die deformierbare Überzugsschicht eine derartige Elastizität hat, daß sie um einen Betrag von einem Prozent (1 %) bis vierzig Prozent (40 %) ihrer ursprünglichen Dicke zusammengedrückt werden kann.

21. Endoprothese nach einem der Ansprüche 14 bis 20, **dadurch gekennzeichnet**, daß die Überzugsschichten eine offene Porengröße von 50 bis 400 Mikromillimeter haben.

## Revendications

1. Endoprothèse pour insertion dans un logement (21) formé dans le canal d'un os (16) d'un patient, comprenant une tige allongée (12) formant une région proximale (13), une région distale (14) et une extrémité distale (15) qui est dotée d'un élément absorbant l'énergie (34) d'un matériau souple, poreux, biocompatible, élastique et déformable s'étendant depuis ladite extrémité, caractérisée en ce que le matériau est un matériau favorisant la croissance interne de tissu d'une épaisseur, mesurée dans la direction axiale de la prothèse, supérieure à 2 millimètres.

2. Endoprothèse selon la revendication 1, caractérisée en ce que ledit élément est fixé à l'extrémité distale (15) de la prothèse.

3. Endoprothèse selon la revendication 1 ou 2, caractérisée en ce que ledit élément (34) a une structure tridimensionnelle avec un faible module d'élasticité, une phase solide biocompatible continue, et une phase fluide biocompatible.

4. Endoprothèse selon la revendication 3, caractérisée en ce que ladite phase fluide biocompatible est continue avec l'environnement ambiant.

5. Endoprothèse selon la revendication 3 ou 4, caractérisée en ce que ledit élément (34) a un comportement de compression, aux contraintes-tensions, d'un degré sensiblement moins élevé que celui de l'os réticulé.

6. Endoprothèse selon l'une quelconque des revendications 1 à 5, caractérisée en ce que ledit matériau a une ouverture de pores comprise entre 50 et 400 micromillimètres.

7. Endoprothèse selon l'une quelconque des revendications 1 à 6, caractérisée en ce que ledit élément a une épaisseur supérieure à 4 mm.

8. Endoprothèse selon l'une quelconque des revendications 1 à 7, caractérisée en ce que ledit matériau a une porosité telle que le volume dudit élément peut rétrécir jusqu'à 60-80 % de son volume original avant que la résistance substantielle à la compression se manifeste.

9. Endoprothèse selon l'une quelconque des revendications 1 à 8, caractérisée en ce que ledit matériau est élastomère.

10. Endoprothèse selon l'une quelconque des revendications 1 à 8, caractérisée en ce que ledit matériau est non-élastomère.

11. Endoprothèse selon l'une quelconque des revendications 1 à 8, caractérisée en ce que ledit matériau comprend des fibres de polytétrafluoroéthylène, en mélange avec des fibres de carbone, du graphite ou de l'oxyde d'aluminium et liées avec une résine de polytétrafluoroéthylène frittée.

12. Endoprothèse selon l'une quelconque des revendications 1 à 11, caractérisée en ce que ledit matériau absorbant l'énergie (34) a la forme d'un coussinet (35) fixé à l'extrémité distale de la tige (12) de la prothèse.

13. Endoprothèse selon l'une quelconque des revendications 1 à 11, caractérisée en ce que ledit matériau absorbant l'énergie (34) est d'un seul tenant avec un manchon (36) monté sur la tige (12) de la prothèse et s'étendant sur l'extrémité (15) de la prothèse.

14. Endoprothèse selon l'une quelconque des revendications précédentes, caractérisée en ce qu'au moins une portion de la région proximale (13) de la tige (12) de la prothèse est revêtue d'une couche de matériau (26,27,38) biocompatible, poreux, non-déformable et favorisant la croissance interne de tissu.

15. Endoprothèse selon la revendication 14, caractérisée en ce que ladite portion est disposée sur la partie antérieure de la région proximale (13) de la tige (12).

16. Endoprothèse selon la revendication 14 ou 15, caractérisée en ce qu'au moins une autre portion de la région proximale (13) de la tige (12) est revêtue d'un matériau (28,36,40) biocompatible, poreux, déformable et favorisant la croissance interne de tissu.

17. Endoprothèse selon l'une quelconque des revendications 1 à 16, caractérisée en ce qu'au moins une portion de la région distale (14) de la tige (12) est revêtue d'un matériau biocompatible, poreux, souple, élastique, déformable, et favorisant la croissance interne de tissu.

18. Endoprothèse selon la revendication 14, 15 ou 16, caractérisée en ce qu'en dehors des régions revêtues dudit matériau non-déformable (26, 27, 38), pratiquement tout le reste de la tige (12) est revêtu d'un matériau biocompatible, poreux, souple, élastique, déformable, et favorisant la croissance interne de tissu.

19. Endoprothèse selon l'une quelconque des revendications 14 à 18, caractérisée en ce que lesdits revêtements ont une épaisseur inférieure à 2,5 mm.

20. Endoprothèse selon l'une quelconque des revendications 16 à 19, caractérisée en ce que la couche de revêtement déformable a une élasticité telle qu'elle peut être comprimée de 1 à 40 % de son épaisseur originale.

21. Endoprothèse selon l'une quelconque des revendications 14 à 20, caractérisée en ce que les couches de revêtement ont une ouverture de pores comprise entre 50 et 400 micro-millimètres.

FIG. 6

FIG. 10

FIG. 11

FIG. 5

FIG. 2

FIG. 3

FIG. 4

FIG. 1
(PRIOR ART)

19

13 26

10a

36

14

15

35

*FIG. 7*

19

27

27

13

10b

14

36

15

35

*FIG. 8*

19

13

28

12

10c

10

10

14

15

35

11 11

*FIG. 9*

FIG. 12

FIG. 13

FIG. 14

FIG. 15